(19) Europäisches Patentamt
European Patent Office
Office européen des brevets

(11) **EP 1 471 943 B1**

(12) **EUROPÄISCHE PATENTSCHRIFT**

(45) Veröffentlichungstag und Bekanntmachung des Hinweises auf die Patenterteilung:
**08.10.2008  Patentblatt 2008/41**

(21) Anmeldenummer: **03704225.6**

(22) Anmeldetag: **17.01.2003**

(51) Int Cl.:
**A61K 47/48** (2006.01)

(86) Internationale Anmeldenummer:
**PCT/DE2003/000124**

(87) Internationale Veröffentlichungsnummer:
**WO 2003/059392 (24.07.2003 Gazette 2003/30)**

(54) **KONJUGAT ZUR BEHANDLUNG PROKARYONTISCHER INFEKTIONEN**

CONJUGATE FOR TREATING PROKARYOTIC INFECTIONS

CONJUGUE POUR TRAITER DES INFECTIONS PROCARYOTES

(84) Benannte Vertragsstaaten:
**AT BE BG CH CY CZ DE DK EE ES FI FR GB GR HU IE IT LI LU MC NL PT SE SI SK TR**

(30) Priorität: **18.01.2002  DE 10201862**

(43) Veröffentlichungstag der Anmeldung:
**03.11.2004  Patentblatt 2004/45**

(73) Patentinhaber: **Deutsches Krebsforschungszentrum Stiftung des öffentlichen Rechts
69120 Heidelberg (DE)**

(72) Erfinder:
• **BRAUN, Klaus
69207 Sandhausen (DE)**
• **BRAUN, Isabell
35091 Cölbe-Bürgeln (DE)**
• **DEBUS, Jürgen
76698 Stettfeld (DE)**
• **PIPKORN, Rüdiger
69124 Heidelberg (DE)**
• **WALDECK, Waldemar
69514 Laudenbach (DE)**

(74) Vertreter: **Schüssler, Andrea et al
Kanzlei Huber & Schüssler
Truderinger Strasse 246
81825 München (DE)**

(56) Entgegenhaltungen:
WO-A-00/68265          WO-A-01/05432
WO-A-96/03519          WO-A-96/11205
WO-A-99/65506          WO-A-03/006065

• PIPKORN, R. ET AL: "Peptide carrier for efficient drug transport into living cells" PEPTIDES: THE WAVE OF THE FUTURE, PROCEEDINGS OF THE SECOND INTERNATIONAL AND THE SEVENTEENTH AMERICAN PEPTIDE SYMPOSIUM, SAN DIEGO, CA, UNITED STATES, JUNE 9-14, 2001 (2001), 931-932. EDITOR(S): LEBL, MICHAL; HOUGHTEN, RICHARD A. PUBLISHER: AMERICA, 2002, XP0008019046
• GOOD L ET AL: "Bactericidal antisense effects of peptide - PNA conjugates" NATURE BIOTECHNOLOGY 2001 UNITED STATES, Bd. 19, Nr. 4, 2001, Seiten 360-364, XP002252283 ISSN: 1087-0156
• NIELSEN P E: "Peptide nucleic acids as therapeutic agents." CURRENT OPINION IN STRUCTURAL BIOLOGY. JUN 1999, Bd. 9, Nr. 3, Juni 1999 (1999-06), Seiten 353-357, XP002252284 ISSN: 0959-440X
• BRAUN KLAUS ET AL: "A biological transporter for the delivery of peptide nucleic acids (PNAs) to the nuclear compartment of living cells." JOURNAL OF MOLECULAR BIOLOGY. 26 APR 2002, Bd. 318, Nr. 2, 26. April 2002 (2002-04-26), Seiten 237-243, XP0002248667 ISSN: 0022-2836

**Beschreibung**

Die vorliegende Erfindung betrifft ein Konjugat zur Behandlung prokaryontischer Infektionen aus einem die prokaryontische Zellmembran passierenden Peptid oder Protein als Transportvermittler und einer gewünschten, in den Prokaryonten einzubringenden Peptid-Nukleinsäure (PNA), die gegen ein Gen des Prokaryonten gerichtet ist, das eine Antibiotikaresistenz verleiht.

Weltweit sind bakterielle Infektionen auf dem Vormasch, die mit den bisher zur Verfügung stehenden Antibiotika aufgrund von Resistenzbildung nicht mehr behandelbar sind. Kritisch ist vor allem die Lage in Kliniken, z.B. auf Intensivstationen, wo täglich Antibiotika verabreicht werden und daher Erreger leicht Resistenzen ausbilden können. Auch gerade der falsche oder zu häufige Gebrauch von Antibiotika hat zu einer raschen Zunahme resistenter Bakterienstämme geführt. Zwar wurde bisher das Problem dadurch gelöst, dass alternative bzw. neuentwickelte Antibiotika eingesetzt wurden (z.B. Cephalosporine/derivatisierte Antibiotika), Kombinationstherapien (z.B. Cotrimoxazol/Sulfonamide), aber auch hier treten in relativ kurzer Zeit neue Resistenzen auf. So ist z.B. auch Vancomycin, bisher vor kurzem noch gegen bestimmte Bakterienstämme wirksam, aufgrund der inzwischen gebildeten Resistenzen oft nicht mehr therapeutisch einsetzbar. Zwar werden zur Entwicklung neuer natürlicher Antibiotika verschiedenen Ursprungs aus Pro- und Eukaryonten sehr große Anstrengungen unternommen, gegen die rasch mutierenden Bakterienstämme konnten jedoch nur temporäre bzw. Teilerfolge erzielt werden.

Konjugate aus dem nicht-bakteriellen Transportpeptid (KFF)$_3$K und einer Peptid-Nukleinsäure, die gegen die mRNA von lacZ gerichtet ist wurden offenbart von Good et al. ("Bactericial antisense effects of peptide-PNA conjugates" Nature Biotechnology 2001, Bd. 19, Nr. 4, Seiten 360-364). Diese Konjugate hemmen das Bakterienwachstum durch einen Antisense-Mechanismus gegen die mRNA von lacZ.

Somit liegt der Erfindung im wesentlichen das technische Problem zugrunde, Mittel bereitzustellen, die eine wirksame Therapie von Erkrankungen ermöglichen, die in Zusammenhang mit gegen Antibiotika resistenten Prokaryonten, z.B. Bakterien stehen.

Die Lösung dieses technischen Problems erfolgte durch die Bereitstellung der in den Patentansprüchen gekennzeichneten Ausführungsformen.

Von den Erfindern wurde zur Erzielung der Lösung des technischen Problems so vorgegangen, dass eine erneute Sensibilisierung der Bakterien gegenüber den (klassischen oder modernen) Antibiotika, gegen die sie resistent waren, durchgeführt wurde. Für diese Sensibilisierung wurde ein Konjugat entwickelt, das die folgenden Komponenten umfaßt: (a) ein Peptid oder Protein als Transportvermittler für die prokaryontische Zellmembran, d.h. vorzugsweise ein per se antibakterielles Peptid (z.B. Defensin), das aufgrund seiner Ladung und Struktur durch Porenbildung die bakterielle Zellwand passieren und auch schädigen kann und (b) die in den Prokaryonten einzubringende, Peptid-Nukleinsäure (PNA), die gegen ein Gen gerichtet ist, das dem Prokaryonten eine Antibiotikaresistenz verleiht. In den zu der vorliegenden Erfindung führenden Beispielen konnte gezeigt werden, dass an Ampicillin/Neomycin resistenten Bakterien (*E. coli*), die mit spezifischen Defensin-Konjugat-PNAs$_{Ampicillin/Neomycin}$ behandelt wurden, eine erfolgreiche Überwindung der Antiobiotikaresistenz erreicht wurde. Somit stellen die erfindungsgemäßen Konjugate eine neue Klasse von Antibiotika dar, die die bakteriellen Abwehrmechanismen überwinden können, bzw. mit deren Hilfe es möglich ist, bereits bekannten klassischen Antibiotika, wie z.B. Benzylpenicillinen, Tetracyclinen und Neomycinen, deren ursprüngliche Wirkungseffizienz wieder zu verleihen. Dieser Weg weicht völlig von den bisherigen Therapie- bzw. Forschungsstrategien ab, ergänzt diese jedoch sehr erfolgversprechend. Die mittels der vorliegenden Erfindung durchführbare (Doppel) strategie ist einerseits spezifisch gegen prokaryontische, z.B. bakterielle Membranen gerichtet und für Prokaryonten toxisch, andererseits lassen sich mit den erfindungsgemäßen Konjugaten gegen Prokaryonten gerichtete Verbindungen einschleusen, z.B. speziell gegen bakterielle Gene gerichtete antisense-Nukleinsäuren (anti-Gen-Nukleinsäuren), z.B. PNAs, Peptid-Domänen, modifizierte Nukleotide, Inhibitoren von Enzymen etc..

Die erfindungsgemäßen Konjugate weisen somit u.a. die folgenden Vorteile auf: (a) Für deren Etablierung wird kein aufwändiges Screening-Verfahren benötigt und (b) in einer besonderen Ausführungsform kann die Revitalisierung klassischer Antibiotika durch Blockieren der prokaryontischen Resistenzmechanismen auf Nukleinsäure-Ebene erzielt werden, wodurch die "alten" Antibiotika, die bisher aufgrund ihrer mangelnden Wirksamkeit oft nicht mehr eingesetzt werden konnten, wieder eine Bedeutung erlangen.

Somit betrifft die vorliegende Erfindung ein Konjugat, das zur Behandlung prokaryontischer Infektionen geeignet ist und die folgenden Komponenten aufweist:

(a) ein die prokaryontische Zellmembran passierendes Peptid oder Protein als Transportvermittler; und
(b) eine in den Prokaryonten einzubringende und gegen diesen gerichtete PNA, die die Transkription eines prokaryontischen Gens hemmt, das eine Antibiotikum-Resistenz verleiht.

Bezüglich Verfahren zur Herstellung der einzelnen Komponenten der Konjugate und zu deren Verknüpfung wird auf die deutsche Patentanmeldung Nr. 199 33 492.7 sowie die nachstehenden Beispiele verwiesen. Die Ankopplung der anderen Bestandteile (z.B. Spacer und/oder PNA) an den Transportvermittler erfolgt jedenfalls durch kovalente chemische Bindung. Gegebenenfalls kann die Einfügung einer Redoxspaltstelle (-S∩S-) zwischen Transportvermittler und einzuschleusender Verbindung auf chemischen Wege über eine Redoxkopplung erfolgen. Auch zwischen einem ggf. vorhandenen Spacer und der einzubringenden PNA liegt eine kovalente Bindung vor, bevorzugt eine Säureamid-Bindung. Mögliche Alternativen sind Ether- oder Ester-Bindungen, je nach den in der zu konjugierenden Verbindung vorhandenen funktionellen Gruppe(n).

Das erfindungsgemäße Konjugat eignet sich zur Behandlung aller prokaryontischer Infektionen, z.B. Infektionen mit Bakterien, vorzugsweise humanpathogenen Bakterien, Mycoplasmen oder Hefen.

Der Transportvermittler des erfindungsgemäßen Konjugats ist ein Peptid oder Protein, das die prokaryontische Zellmembran passieren und die gewünschte Verbindung in das Zytoplasma einschleusen kann. Die Länge dieses Peptids bzw. Proteins unterliegt keiner Beschränkung, solange es die obige Eigenschaft aufweist. Hergestellt wird der ausgewählte Transportvermittler vorzugsweise auf biologischem Weg (Reinigung natürlicher Transportvermittlerproteine/peptide oder Klonierung und Expression der Sequenz in einem eukaryotischen oder prokaryotischen Expressionssystem), bevorzugt aber auf synthetischem Weg, z.B. nach dem Merrifield-Verfahren (Merrifield, J. Am. Chem. Soc. 85 (1963), 2149) .

Vorzugsweise handelt es bei diesem Transportvermittler um eine Verbindung, die per se bereits für den Prokaryonten schädlich ist, z.B. dadurch, dass sie zu einer Membranschädigung (z.B. durch Bildung von Poren oder Läsionen) führt. Dabei handelt es sich vorzugsweise um Defensine oder Holine (Bakteriophagen-Proteindomänen).

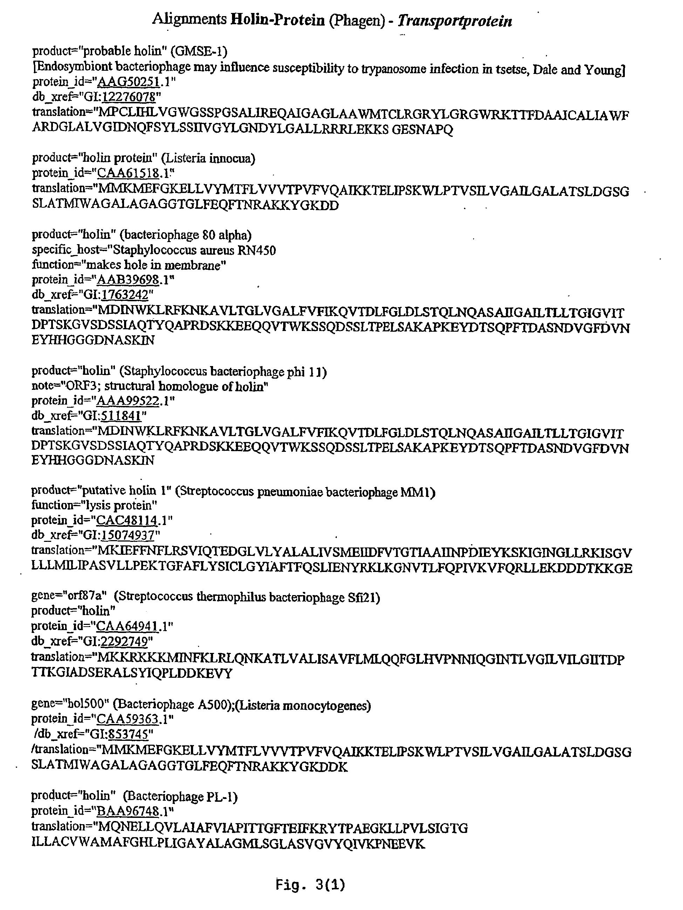

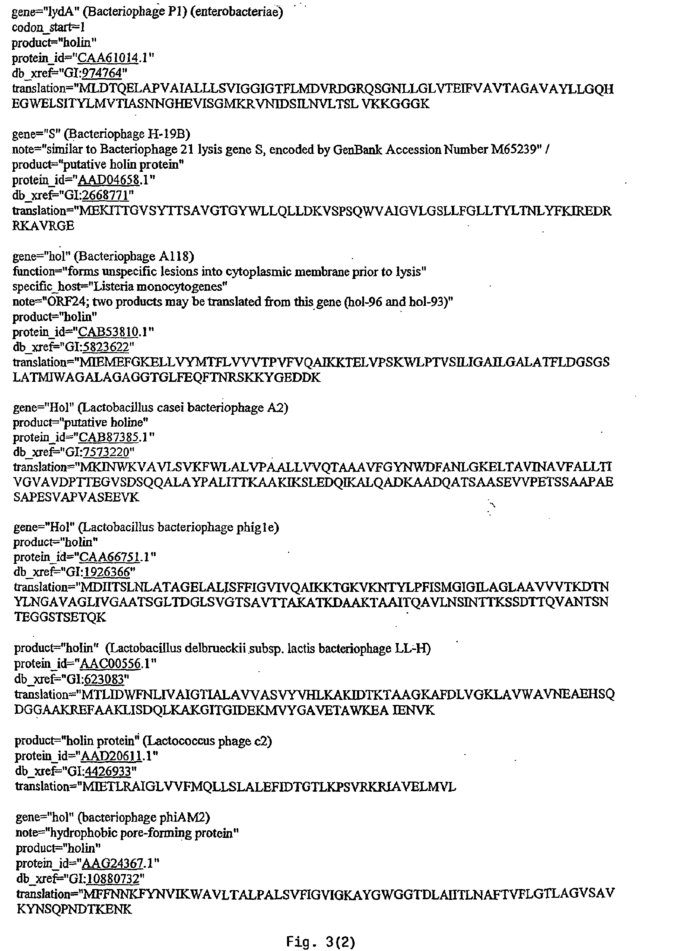

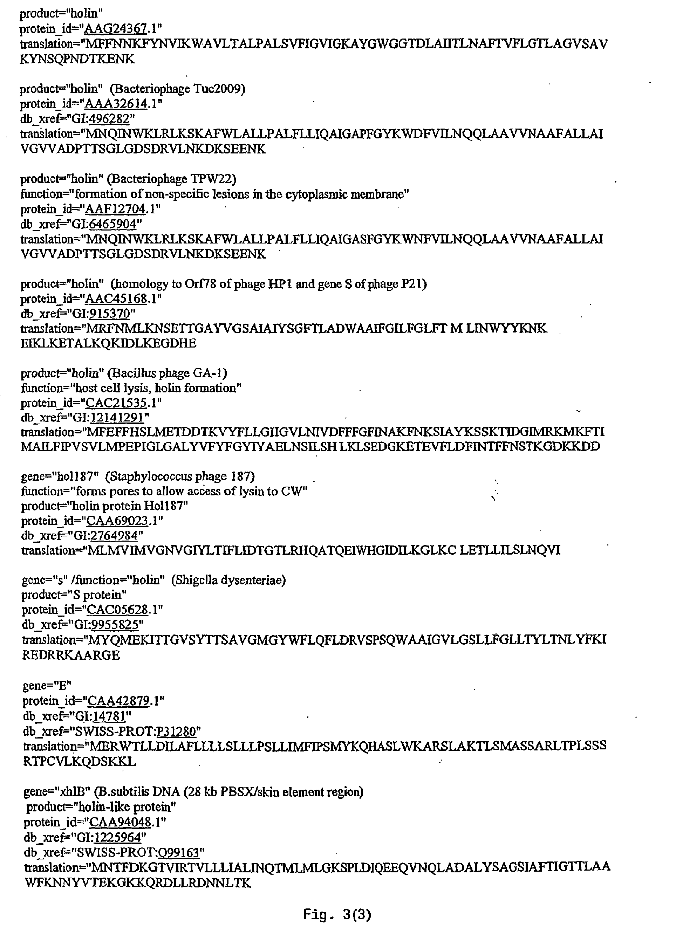

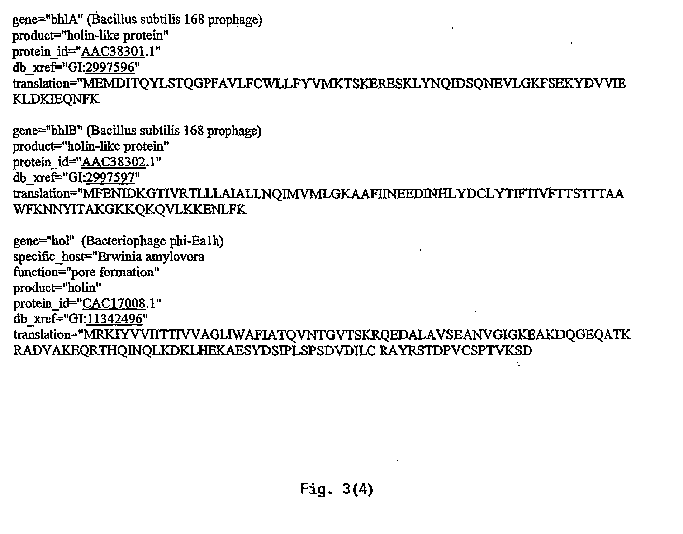

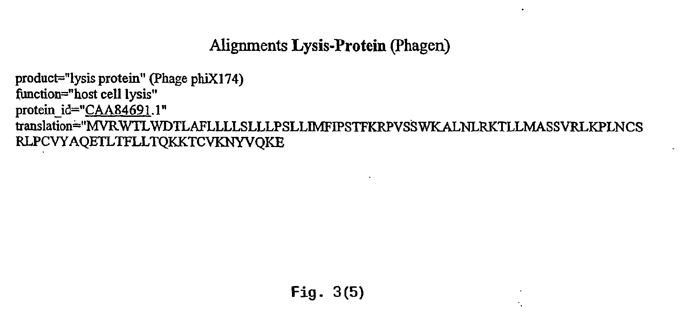

In einer besonders bevorzugten Ausführungsform umfaßt das erfindungsgemäße Konjugat einen Transportvermittler, der ein Phagen-Holin-Protein umfaßt, wobei ein Phagen-Holin-Protein mit einer der in Figur 3 dargestellten Aminosäuresequenzen oder Fragmente oder Varianten davon, die noch die prokaryontische Zellmembran passieren können, noch mehr bevorzugt sind.

Die in der vorliegenden Erfindung verwendeten Begriffe "Variante" oder "Fragment" umfassen Proteine/Peptide mit Aminosäuresequenzen, die sich gegenüber den in Figur 3 angegebenen Sequenzen durch Deletion(en), Insertion (en), Austausch(e) von Aminosäureresten und/oder andere im Stand der Technik bekannte Modifikationen unterscheiden bzw. ein Fragment des ursprünglichen Proteins/Peptids umfassen, wobei die Varianten bzw. Fragmente des Proteins/Peptids im Wesentlichen noch die biologischen Eigenschaften des Ausgangsproteins/peptids aufweisen, d.h. die prokaryontische Zellmembran passieren können. Verfahren zur Erzeugung der vorstehenden Änderungen in der Aminosäuresequenz sind dem Fachmann bekannt und in Standardwerken der Molekularbiologie beschrieben, beispielsweise in Sambrook et al., Molecular Cloning: A Laboratory Manual, 2. Ausgabe, Cold Spring Harbor Laboratory Press, Cold Spring Harbor NY (1989)). Der Fachmann ist auch in der Lage zu bestimmen, ob ein solches Protein bzw.

Peptid noch über die gewünschten biologischen Eigenschaften verfügt, beispielsweise mit den in den nachstehenden Beispielen beschriebenen Verfahren.

In einer alternativen bevorzugten Ausführungsform des erfindungsgemäßen Konjugats umfaßt der Transportvermittler ein Defensin, vorzugsweise ein humanes Defensin. Bei den Defensinen handelt es sich um Polypeptide bzw. Peptide mit antimikrobieller Wirkung, die wichtige Faktoren bei der angeborenen Immunität von Vertebraten und Nicht-Vertrebaten darstellen. Defensine wurden z.B. aus Tieren (einschl. dem Menschen), Pflanzen und Insekten isoliert. Sie bestehen in der Regel aus 29 bis 42 Aminosäuren und enthalten drei Disulfidbrücken, die von drei Cysteinpaaren gebildet werden. Für die vorliegenden Erfindung geeignete Defensine sind z.B. in Tang et al., Science 286 (1999), 498; Saido-Sakanaka et al., Biochem. J. 338 (1999), 29; Raj et al., Biochem J. 347 (2000), 633; Yu et al., J. Biol. Chem. 275 (No.6) (2000), 3943 beschrieben.

Die in den Prokaryonten einzubringende Verbindung ist eine gegen die Expression eines Gens gerichtete Peptid-Nukleinsäure (PNA). Der Einsatz der protease- und nukleaseresistenten Peptid-Nukleinsäuren ("PNAs"), bei denen es sich um Oligonukleotid-Derivate handelt, bei denen das Zuckerphosphat-Rückgrat bevorzugt durch Ethyl-Amin verbundene α-Amino-Ethyl-Glycin-Einheiten substituiert ist, erlaubt aufgrund ihrer physiko-chemischen Eigenschaften unter physiologischen Bedingungen eine stabile und effiziente Blockierung der Transkription der gewünschten Gene. Mit diesen PNAs wird eine auf dem Antisense-Prinzip basierende Anti-Gen-Strategie verfolgt, bei der jedoch nicht die mRNA, sondern das Gen selbst das Ziel ist. Dabei hybridisieren die PNAs über die Bildung einer Triple-Helix an die Ziel-DNA. Der Zielbereich kann einerseits ein transkribierter Bereich des zu blockierenden Gens, z.B. des eine Antibiotikaresistenz verleihenden Gens sein, andererseits ein regulatorischer Bereich, dessen Blockierung über die PNAs ebenfalls die Transkription hemmt. Geeignete Bereiche können vom Fachmann anhand der bekannten DNA-Sequenzen bzw. deren Funktion identifiziert werden. Vorzugsweise weisen die Peptid-Nukleinsäuren eine Länge von mindestens 15 Basen auf, besonders bevorzugt sind Peptid-Nukleinsäuren mit einer Länge von mindestens 18 Basen, ganz bevorzugt von mindestens 21 Basen und am meisten bevorzugt von mindestens 23 Basen. Die Peptid-Nukleinsäure kann ggf. auch markiert sein, z.B. radioaktiv, mit einem Farbstoff, mit Biotin/Avidin usw. Die Synthese von PNAs ist dem Fachmann bekannt und z.B. auch in Nielsen et al., Science 254 (1991), 1497-1500, beschrieben. Der hier verwendete Begriff "Gen", umfaßt nicht nur Gene des Genoms des Prokaryonten, sondern auch Gene auf extra-genomischen Elementen, z.B. Plasmiden etc..

Die Peptid-Nukleinsäure (PNA) ist gegen ein Gen gerichtet, das eine Antibiotikaresistenz verleiht, vorzugsweise ist die Antibiotikaresistenz eine Penicillin-, Ampicillin-, Kanamycin- oder Tetracyclin-Resistenz.

Weiter kann das Konjugat ggf. einen Spacer enthalten, der sich zwischen dem Transportvermittler und der Peptid-Nukleinsäure (PNA) befindet. Der Spacer dient dazu, ggf. vorhandene sterische Wechselwirkungen zwischen den Komponenten aufzuheben bzw. günstig zu beeinflussen.

Die Struktur des erfindungsgemäßen Konjugats ist vorzugsweise: Transportvermittler-Spacer-PNA. Besonders bevorzugt sind die Spacer Polylysin, Polyglycin oder Poly(Glycin/Lysin). Für die erfindungsgemäßen Zwecke liegt die Länge des Spacers vorzugsweise in einem Bereich von 2 bis 6 Aminosäuren. Vorzugsweise ist der Spacer über eine spaltbare Disulfidbrücke (-S∩S-) mit dem Transportvermittler verknüpft.

In einer besonders bevorzugten Ausführungsform des erfindungsgemäßen Konjugats umfaßt die Peptid-Nukleinsäure (PNA) die folgende Sequenz: ATTGTTAGATTTCAT (Orientierung: N-Terminus/Sequenz/C-Terminus).

Die vorliegende Erfindung betrifft schließlich auch ein erfindungsgemäßes Konjugat, gegebenenfalls zusammen mit einem geeigneten Träger, enthaltendes Arzneimittel. Vorzugsweise enthält das Arzneimittel außerdem ein Antibiotikum, für das der Prokaryont durch Verabreichung des Konjugats sensibilisiert wurde. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc.. Die Verabreichung der Arzneimittel erfolgt vorzugsweise parenteral, transdermal oder subcutan. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Infektion, der Art der Verabreichung etc..

Schließlich betrifft die vorliegende Erfindung die Verwendung eines erfindungsgemäßen Konjugats zur Herstellung eines Medikaments zur Behandlung einer prokaryontischen Infektion, wobei diese Infektion vorzugsweise von einem Prokaryonten verursacht ist, der eine Resistenz gegenüber einem Antibiotikum aufweist.

Legenden zu den Figuren:

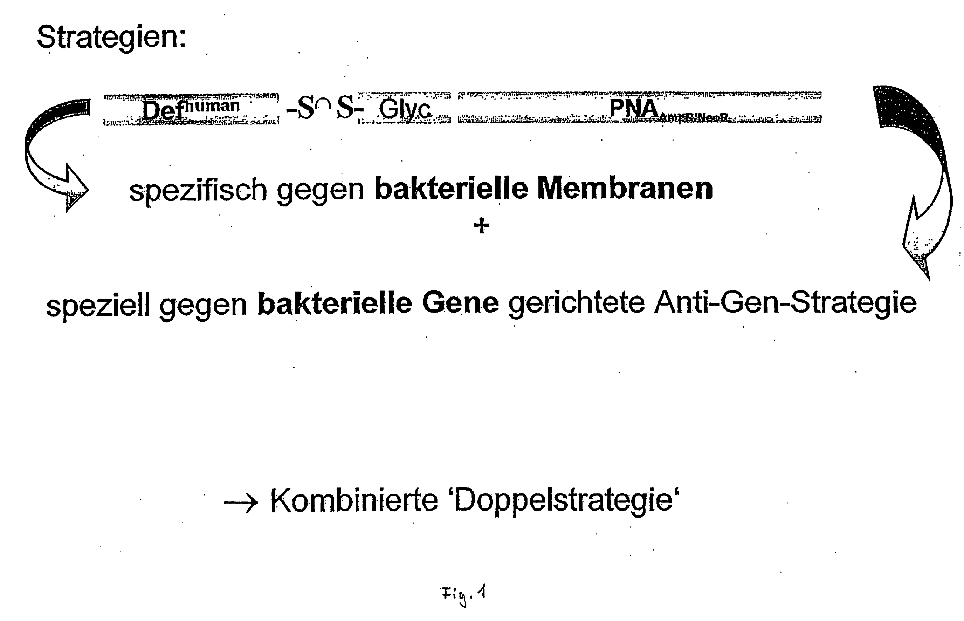

Figur 1: Schematische Darstellung eines erfindungsgemäßen Konjugats mit Defensin als Transportvermittler und einer gegen eine Ampicillin- bzw. Kanamycin-Resistenz gerichteten PNA

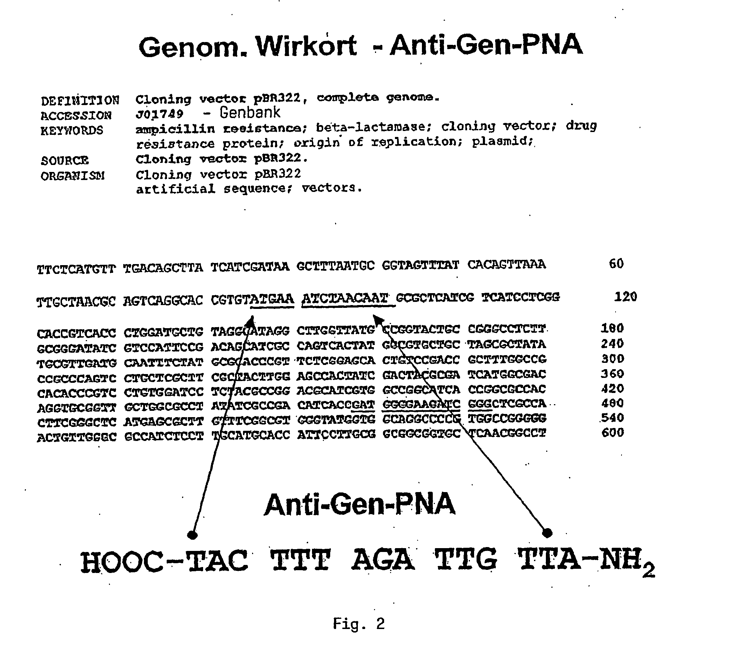

Figur 2: Darstellung eines Teilbereichs der pDNA von Plasmid pBR322 einschl. der für die Konstruktion eines PNA-Konjugats verwendeten Sequenzen
Die Sequenz der gegen eine Ampicillin-Resistenz gerichteten Anti-Gen-PNA ist in der Figur unten gezeigt. Der unterstrichene Bereich aus der beta-lactamase kodierenden pDNA-Sequenz von pBR322 entspricht dem Zielbereich für das PNA-Konjugat.

Figur 3: Zusammenstellung von Holin-Protein-Sequenzen, die als Transportvermittler in den erfindungsgemäßen Konjugaten geeignet sind.
Die Aminosäurensequenzen der 28 einzelnen Holine sind im Einbuchstaben-Code dargestellt.

Figur 4: Ergebnisse der Behandlung von (durch Transformation mit pBR322 gegen Ampicillin bzw. mit pEGFP-N$_1$ (Clontech, Deutschland, Heidelberg) gegen Kanamycin resistenten kompetenten *E.coli* mit oder ohne (Kontrolle) den erfindungsgemäßen Konjugaten
Obere Reihe: Kanamycin-Nährboden + Kanamycin-Resistenz-Konjugat (500 nM); untere Reihe: Ampicillin-Agar + Ampicillin-Resistenz-Konjugat (500 nM); linke Spalte: Kontrollen (ohne PNA-Konjugate). Ein deutlicher Effekt der erfindungsgemäßen Konjugate (Wiedererlangung der Antibiotika-Sensitivität) ist zu beobachten.

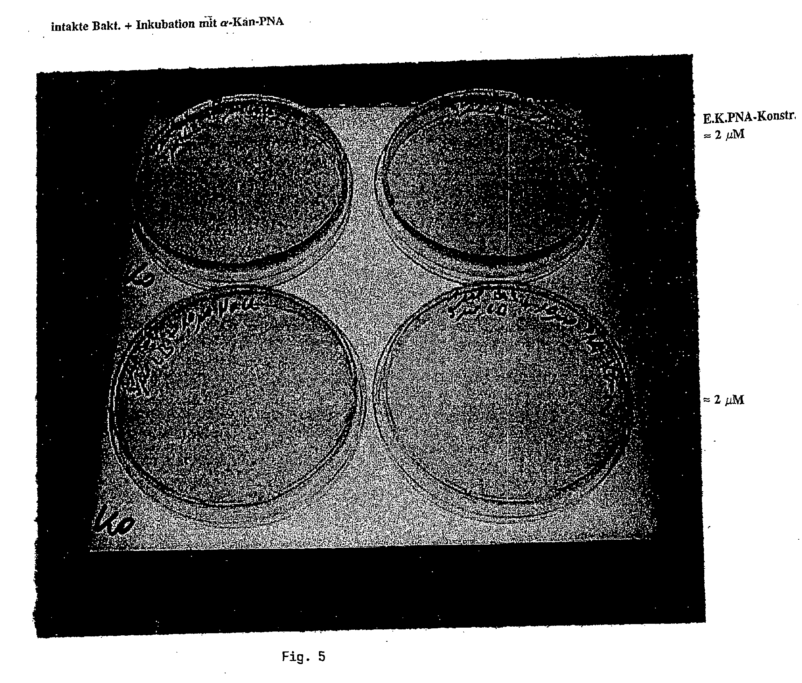

Figur 5: Ergebnisse der Behandlung von gegen Kanamycin resistenten intakten (nicht-kompetenten) *E.coli* mit oder ohne (Kontrolle) den erfindungsgemäßen Konjugaten
Obere Reihe: Kanamycin-Nährboden + Kanamycin-Resistenz-Konjugat (2 μM); untere Reihe: Kanamycin-Nährboden + Kanamycin-Resistenz-Konjugat (20 μM); linke Spalten: Kontrollen (ohne PNA-Konjugate). Ein deutlicher Effekt der erfindungsgemäßen Konjugate (Wiedererlangung der Antibiotika-Sensitivität) ist bereits bei einer Konzentration von 2μM zu beobachten, bereits bei 20μM werden alle Bakterien wieder sensibilisiert, d.h. abgetötet.

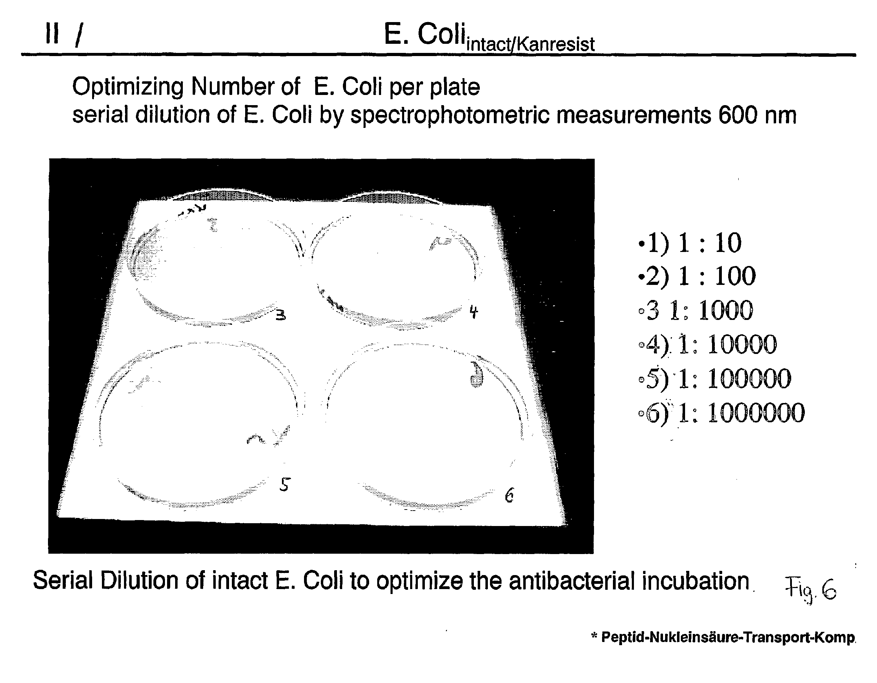

Figur 6: Ergebnisse der Behandlung von gegen Kanamycin resistenten intakten (nicht-kompetenten) *E.coli* mit verschiedenen Konzentrationen der erfindungsgemäßen Konjugate zur Ermittlung der optimalen Bakterienkonzentration zur Quantifizierung

Es wurden Kanamycin-Nährböden verwendet, auf die resistente Bakterien ausplattiert wurden. Zur Ermittlung der optimalen Bakterienkonzentration wurde eine Verdünnungsreihe mit dem Kanamycin-Resistenz-Konjugat in abnehmenden Zehnerpotenzen verwendet.

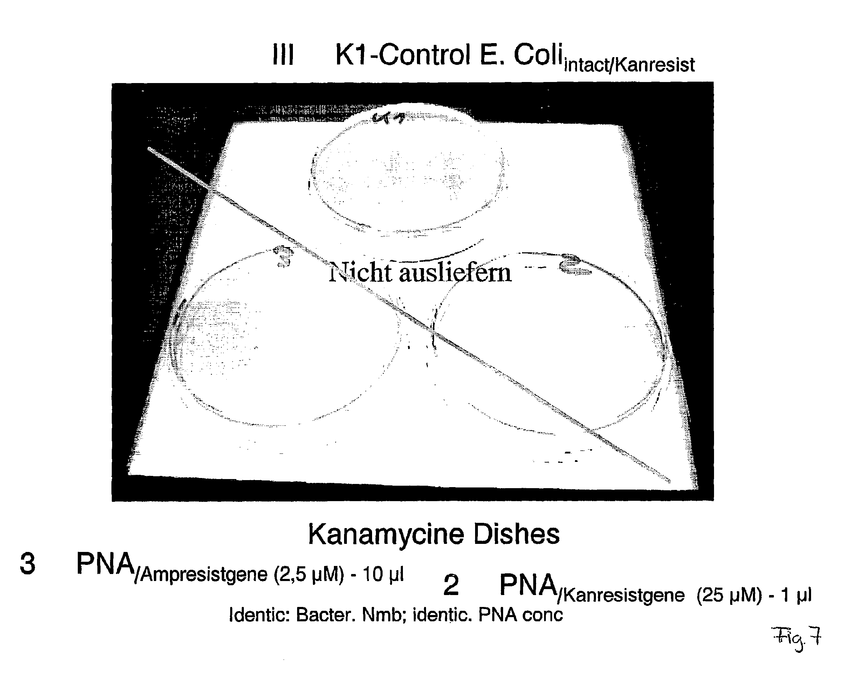

Figur 7: Ergebnisse der Behandlung von gegen Kanamycin bzw. Ampicillin resistenten intakten (nicht-kompetenten) *E.coli* mit den erfindungsgemäßen Konjugaten
Platte K1: Kontrolle, Platte 2: 250 nM Kanamycin-Resistenz-Konjugat; Platte 3: 250 nM Ampicillin-Resistenz-Konjugat. Die Ergebnisse zeigen Bakterienhemmung für Ampicillin und Kanamycin bei 250 nM Konjugat.

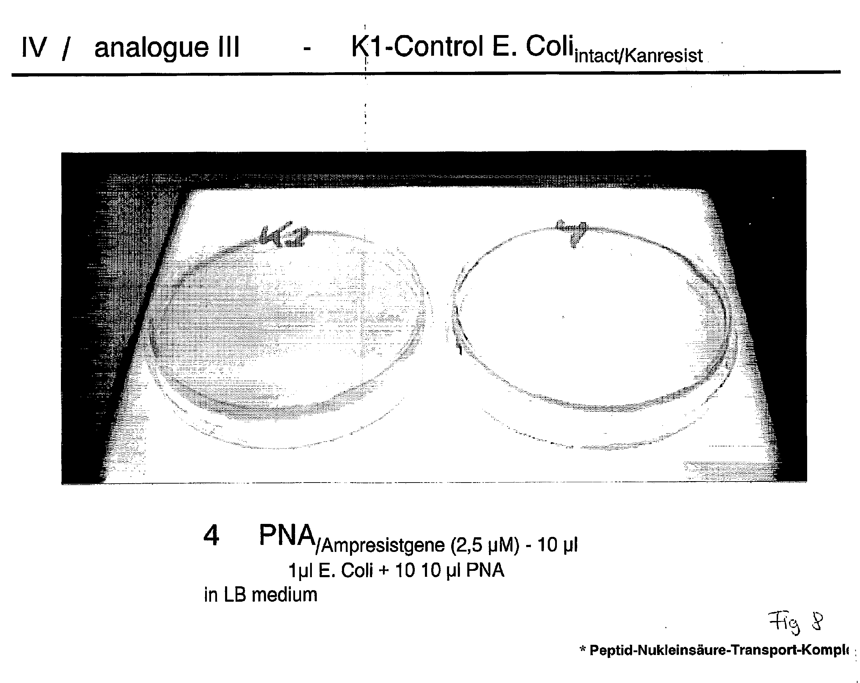

Figur 8: Ergebnisse der Behandlung von gegen Ampicillin resistenten intakten (nicht-kompetenten) *E.coli* mit den erfindungsgemäßen Konjugaten
wie bei Figur 7; K2: Kontrolle, Platte 4: 250 nM Ampicillin-Resistenz-Konjugat.

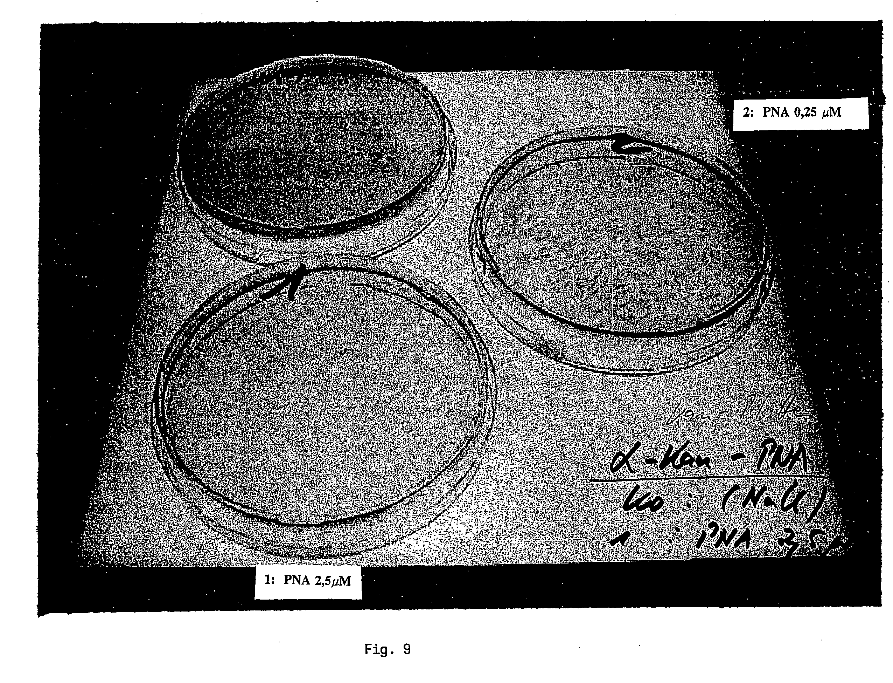

Figur 9: Ergebnisse der Behandlung von gegen Kanamycin resistenten intakten (nicht-kompetenten) *E.coli* mit den erfindungsgemäßen Konjugaten
wie bei Figur 6; K1: Kontrolle; Platte 1: 2,5 $\mu$M Kanamycin-Resistenz-Konjugat; Platte 2: 250 nM Kanamycin-Resistenz-Konjugat.

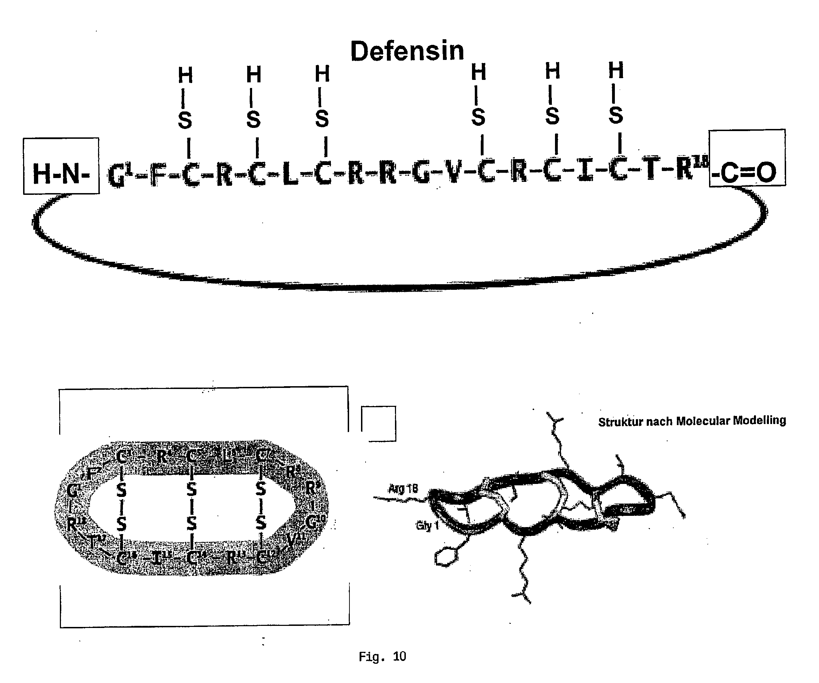

Figur 10: Struktur des für die erfindungsgemäßen Konjugate verwendeten Defensins nach Zyklisierung (oben) und nach Bildung dreier Disulfidbrücke (links unten)
Die hypothetische räumliche Struktur ist rechts unten dargestellt.

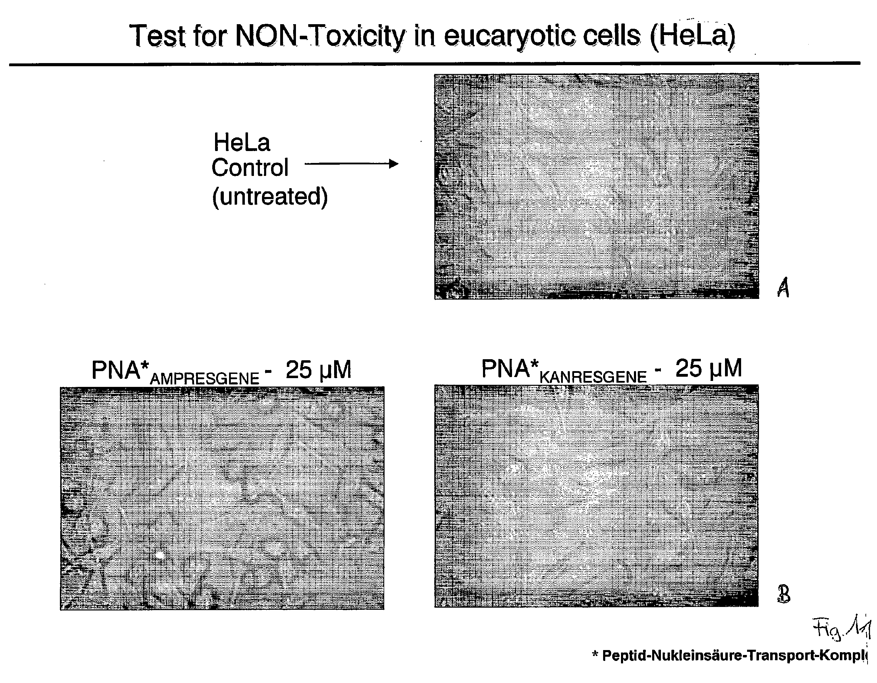

Figur 11: Ergebnisse der Behandlung von HeLa-Zellen mit einem erfindungsgemäßen Konjugat

A: unbehandelte Kontrolle
B: Mit dem erfindungsgemäßen Konjugat behandelte Zellen

Die Erfindung wird weiter anhand der nachfolgenden Beispiele beschrieben.

**Beispiel 1**

**Allgemeine Verfahren**

(A) Zellkultur

Die Bakterien wurden auf Agar mit LB-Medium (und den entsprechenden Antibiotika) ausplattiert und ü.N. bei 37°C inkubiert. HeLa-Zellen wurden in Flüssigkultur gemäß üblichen Bedingungen gezüchtet.

(B) PNA-Synthese

Peptidnukleinsäure (PNA) imitiert eine DNA und wurde ursprünglich als Reagens zur sequenzspezifischen Erkennung doppelsträngiger DNA über eine konventielle Tripelhelix-Bildung entwickelt. Für die Festphasensynthese wurde die Fmoc-Strategie mittels einem vollautomatisierten Synthesegerät (Syro II, Multisyntech, Witten, Deutschland) verwendet. Die Synthese wurde an einem 0,05 mmol Fmoc-AS-Polystyrenharz (1% quervernetzt) durchgeführt. Als Kopplungreagenz wurde 2-(1H-Benzotriazol-1-yl)-1,1,3,3-Tetramethyluroniumhexafluorphosphat (HBTU) verwendet. Die Seitenketten schützenden Gruppen waren Lys(Boc), Asp(Obut), Ser(But), Cys(Trt) und Asn(Trt). Das geschützte Peptidylharz wurde mit 20% Piperidin in Dimethylformamid behandelt. Die Spaltung und Abspaltung der Schutzgruppen wurde durch Behandlung mit 90% Trifluoressigsäure, 5% Äthandithiol, 2,5% Thioanisol und 2,5% Phenol (Vol./Vol./Vol.) für 2,5 Stunden bei Raumtemperatur erzielt. Alle Produkte wurden in Äther präzipitiert und über präparative HPLC (Shimazu LC-8A, Shimazu, Duisburg, Deutschland) auf einer YMC ODS-A 7A S-7 $\mu$m Umkehrphasen-HPLC-Säule (20 x 250 mm) unter Verwendung von 0,1% Trifluoressigsäure in Wasser (A) und 60% Acetonitril in Wasser (B) als Elutionsmittel gereinigt. Die Peptide wurde mit einem linearen Gradienten von 25% B bis 60% B innerhalb von 40 Min. bei einer Durchflußrate von 10 ml/Min. eluiert. Die dem gereinigten Konjugat entsprechenden Fraktionen wurden lyophilisiert. Sequenzen von Einzelmolekülen sowie das vollständige bimodulare Konstrukt wurden über analytische HPLC (Shimadzu LC-10) und Laser-Desorptionsmassenspektroskopie (Finnigan Vision 2000, Finnigan MAT, San Jose, CA, USA) wie nachstehend angegeben charakterisiert.
Die Sequenz der gegen eine Ampicillin-Resistenz gerichteten PNA war wie folgt: $H_2$N-ATTGTTAGATTTCAT-COOH. Dabei handelt es sich um eine Sequenz, die mit dem Bereich von Pos.86 bis Pos.100 der pDNA von pBR322 (GeneBank-Zugangs-Nr. J01749) hybridisieren kann. Die Sequenz der gegen Kanamycin-Resistenz verwendeten PNA war $H_2N$-TCTTGTTCAATCAT-COOH.

(C) Chemische Synthese von Defensin

Für die Festphasensynthese von Defensin (vgl. Figur 10 hinsichtlich der Aminosäuresequenz und Struktur) wurde die Fmoc-Strategie (Merrifield, J.Amer.Chem.Soc. 85 (1963), 2149-2154; Ruegg und Rudinger, Methods Enzymol. 47 (1977), 111-126) mit einem vollautomatischen Synthesegerät (ABI 431; Applied Biosystems, Deutschland, Darmstadt) verwendet. Die Synthese wurde an einem 0,05 mmol Fmoc-Arg(Pbf)-Polystyrenharz (1% quervernetzt) durchgeführt. Als Kopplungreagenz wurde 2-(1H-Benzotriazol-1-yl)-1,1,3,3-tetramethyluroniumhexafluorphos-phat (HBTU) verwendet. Die Seitenketten schützenden Gruppen waren: Thr(But), Arg(Pbf). Für Cys wurden drei verschiedene selektiv spaltbare Schutzgruppen verwendet. Für Cys(3,15) wurde t-Butylthio verwendet, für Cys(5,13) Acetamidomethyl und für Cys(7,11) eine Tritylgruppe.

Im ersten Schritt wurde die t-Butylthio-Schutzgruppe mit Tris(2-Carboxyethyl)phosphin (TCEP) gespalten und die Schwefelbrücke mit 20% DMSO in Wasser gespalten. Im zweiten Schritt wurde die Acetamidomethyl-Schutzgruppe gespalten und zur gleichen Zeit die zweite Schwefelbrücke mit einer 0,01 mol Jod-Lösung oxidiert. Das geschützte Peptidylharz wurde 12 Min. mit 20% Piperidin in Formamid behandelt und danach gründlich mit Dimethylformamid gewaschen. Die Spaltung und Entfernung der Schutzgruppen vom Peptidharz erfolgte durch Behandlung mit 90% Trifluoressigsäure, 5% Ethandithiol, 2,5% Thioisanol und 2,5% Phenol (V./V./V./V.) für 2,5 Stunden bei Raumtemperatur. Das Produkt wurde in Äther präzipitiert. Das Rohmaterial wurde über präparative HPLC (Shimazu LC-8A, Shimazu, Duisburg, Deutschland) auf einer YMC-Pack ODS-A,S-5μ m Umkehrphasen-HPLC-Säule (20 x 150 mm) unter Verwendung von 0,1% Trifluoressigsäure in Wasser (A) und 60% Acetonitril in Wasser (B) als Elutionsmittel gereinigt. Das Peptid wurde mit einem linearen Gradienten von 25% B bis 60% B innerhalb von 40 Min. bei einer Durchflußrate von 20 ml/Min. eluiert. Die dem gereinigten Peptid entsprechenden Fraktionen wurden lyophilisiert.

Als letzter Schritt wurden eine Kopf/Schwanz-Zyklisierung mit Propanphosphonicacid-anhydrid (T3P) durchgeführt und das Reinigungsverfahren wiederholt. Das gereinigte Material wurde über analytische HPLC (Shimadzu LC-10) und Laser-Desorptionsmassenspektroskopie (Finnigan Vision 2000, Finnigan MAT, San Jose, CA, USA) charakterisiert.

Peptidreinigung:

Gradient: analyt. 5%→80% (in einem Zeitraum von 35 Min.);
präparativ: 5%→80% (in einem Zeitraum von 40 Min.);
Reinheit: >90%.

(D) Verknüpfungsreaktionen

Die Verknüpfungreaktionen erfolgten wie in der deutschen Patentanmeldung Nr. 199 33 492.7 beschrieben unter milden oxidativen Bedingungen (DMSO/$H_2O$). Dazu wurden Cysteingruppen des Defensins und des Spacers H-S-Gly und der PNA in einem Bereich von 2 mg/ml in einer 20% DMSO/Wasser-Lösung oxidiert. Die Reaktion war nach etwa 5 Stunden vollständig abgelaufen. Das Fortschreiten der Oxidation wurde über eine analytische C18 Umkehrphasen-HPLC überwacht (Tam et al., J.Amer.Chem.Soc. 113 (1991)). Die Komponenten wurden nach der Merrifield-Methode verknüpft (Merrifield, J. Americ. Chem. Soc. 85 (1963), 2149).

Das so synthetisierte PNA-Modul weist folgende Struktur auf:

$$\mathrm{DEFENSIN_{human}\text{-}S{\cap}S\text{-}(Gly)X\text{-}PNA_{AmpR/NeoR}}$$

$$X = 1\text{-}5$$

Die Aufreinigung erfolgte mittels Umkehrphasen-HPLC, anschließend wurde lyophilisiert. Nach Bestimmung der Masse mittels MS wurde das Lyophilisat in einem definiertem Volumen physiologischer Kochsalzlösung zu einer Stammlösung von 10 μM gelöst.

**Beispiel 2**

**Bestimmung der Wirksamkeit der erfindungsgemäßen Konjugate bei antibiotikaresisten *E.coli*-Stämmen**

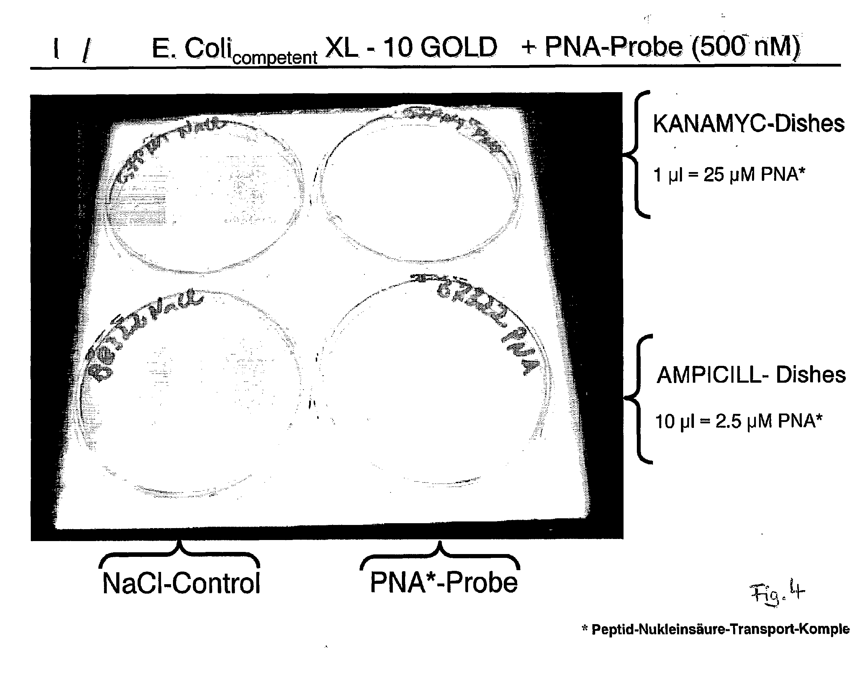

Resistente Bakterien wurden auf übliche Agarplatten mit und ohne Antiobiotikum konfluent ausplattiert und zum Teil mit den erfindungsgemäßen Konjugaten behandelt. In einem Vorexperiment wurden bereits kompetente *E.coli* (d.h. mit bereits durchlöcherten Membranen) verwendet; siehe Figur 4; Obere Reihe: Kanamycin-Nährboden + Kanamycin-Resistenz-Konjugat (500 nM); untere Reihe: Ampicillin-Agar + Ampicillin-Resistenz-Konjugat (500 nM); linke Spalten: Kontrollen. Ein deutlicher Effekt der erfindungsgemäßen Konjugate (Wiedererlangung der Antibiotika-Sensitivität) ist zu beobachten.

In den darauf folgenden (in den Figuren 5 bis 9 dargestellten) Experimenten wurden intakte (nicht-kompetente) *E.coli* verwendet und die gegen eine Antibiotikumresistenz gerichteten Konjugate in unterschiedlichen Verdünnungen getestet. Hinsichtlich der verwendeten Bakterien, Antibiotika und Konzentrationen wird auf die Legenden der Figuren verwiesen. Jedenfalls zeigen die Ergebnisse dieser Untersuchungen deutlich, dass die erfindungsgemäßen Konjugate mit einer PNA, die gegen ein Gen gerichtet ist, das eine Anitbiotikaresistenz verleihen kann, eine erneute Sensibilisierung des Bakteriums für das betreffende Antibiotikum bewirken und diese somit mit diesem Antibiotikum wieder bekämpft werden können.

Die nicht-toxische Wirkung dieses Konjugats auf eukaryontische Zellen (HeLa-Zellen) konnte durch Inkubation der Zellen mit/ohne Konjugat (in Konzentrationen wie für die Experimente mit Bakterien) nachgewiesen werden; siehe Figuren 11A+11B.

**Patentansprüche**

1. Konjugat, das zur Behandlung prokaryontischer Infektionen geeignet ist und die folgenden Komponenten aufweist:

    (a) ein Peptid oder Protein, das die prokaryontische Zellmembran passieren kann als Transportvermittler; und
    (b) eine in den Prokaryonten einzubringende Peptid-Nukleinsäure (PNA), die die Transkription eines prokaryontischen Gens hemmt, das eine Antibiotikum-Resistenz verleiht.

2. Konjugat nach Anspruch 1, wobei der Prokaryont ein Bakterium ist.

3. Konjugat nach Anspruch 2, wobei das Bakterium ein humanpathogenes Bakterium ist.

4. Konjugat nach einem der Ansprüche 1 bis 3, wobei das Peptid oder Protein ein antibakterielles Peptid oder Protein ist .

5. Konjugat nach einem der Ansprüche 1 bis 4, wobei der Transportvermittler ein Phagen-Holin-Protein umfaßt, das eine der in Figur 3 dargestellten Aminosäuresequenzen umfaßt oder ein Fragment oder eine Variante davon, das (die) die prokaryontische Zellmembran passieren kann.

6. Konjugat nach einem der Ansprüche 1 bis 4, wobei der Transportvermittler ein Defensin umfaßt.

7. Konjugat nach einem der Ansprüche 1 bis 6, wobei die Antibiotikum-Resistenz eine Penicillin-, Ampicillin-, Kanamycin- oder eine Tetracyclin-Resistenz ist.

8. Konjugat nach einem der Ansprüche 1 bis 7, das folgende Struktur hat: Transportvermittler-Spacer-Peptid-Nukleinsäure (PNA).

9. Konjugat oder Konjugat-Gemisch nach Anspruch 8, wobei der Spacer Polylysin, Polyglycin oder Poly(Glycin/Lysin) ist.

10. Konjugat nach Anspruch 8 oder 9, wobei der Spacer über eine spaltbare Disulfidbrücke mit dem Transportvermittler verknüpft ist.

11. Konjugat nach einem der Ansprüche 6 bis 10, wobei die Peptid-Nukleinsäure die Sequenz $H_2N$-ATTGTTAGATT-TCAT-COOH umfaßt.

12. Arzneimittel, ein Konjugat nach einem der Ansprüche 1 bis 11 enthaltend.

**13.** Arzneimittel nach Anspruch 12, außerdem mindestens ein Antibiotikum enthaltend, für das der Prokaryont durch die Verabreichung des Konjugats wieder sensibilisiert wurde.

**14.** Verwendung eines Konjugats nach einem der Ansprüche 1 bis 11 oder der in Anspruch 13 definierten Zusammensetzung zur Herstellung eines Arzneimittels zur Behandlung einer prokaryontischen Infektion.

**15.** Verwendung nach Anspruch 14, wobei die prokaryontische Infektion durch einen Prokaryonten verursacht ist, der eine Resistenz gegenüber mindestens einem Antibiotikum aufweist.

**Claims**

**1.** A conjugate suited for treating prokaryotic infections and comprising the following components:

(a) a peptide or protein which can penetrate the prokaryotic cell membrane as transport mediator; and
(b) a peptide nucleic acid (PNA) to be introduced into the prokaryote and which inhibts the transcription of a prokaryotic gene giving antibiotic resistance.

**2.** The conjugate according to claim 1, wherein the prokaryote is a bacterium.

**3.** The conjugate according to claim 2, wherein the bacterium is a bacterium pathogenic for humans.

**4.** The conjugate according to any of claims 1 to 3, wherein the peptide or protein is an antibacterial peptide or protein.

**5.** The conjugate according to any of claims 1 to 4, wherein the transport mediator comprises a phage-holin protein comprising one of the amino acid sequences shown in figure 3 or a fragment or variant thereof, which can penetrate the prokaryotic cell membrane.

**6.** The conjugate according to any of claims 1 to 4, wherein the transport mediator comprises a defensin.

**7.** The conjugate according to any of claims 1 to 6, wherein the antibiotic resistance is a resistance to penicillin, ampicillin, kanamycin or tetracycline.

**8.** The conjugate according to any of claims 1 to 7, which has the following structure: transport mediator-spacer-peptide nucleic acid (PNA)

**9.** The conjugate or conjugate mixture according to claim 8, wherein the spacer is polylysine, polyglycine or poly (glycine/lysine).

**10.** The conjugate according to claim 8 or 9, wherein the spacer is linked to the transport mediator via a cleavable disulfide bridge.

**11.** The conjugate according to any of claims 6 to 10, wherein the peptide nucleic acid comprises the sequence $H_2N$-ATTGTTAGATTTCAT-COOH.

**12.** A medicament containing a conjugate according to any of claims 1 to 11.

**13.** The medicament according to claim 12, further containing at least one antibiotic for which the prokaryote was re-sensitized by administering the conjugate.

**14.** Use of a conjugate according to any of claims 1 to 11 or the composition defined in claim 13 for the preparation of a medicament for treating a prokaryotic infection.

**15.** Use according to claim 14, wherein the prokaryotic infection is caused by a prokaryote which is resistant to at least one antibiotic.

**EP 1 471 943 B1**

**Revendications**

1. Conjugué convenant pour le traitement d'infections procaryotes, présentant les composants suivants :

    (a) un peptide ou une protéine qui peut traverser la membrane de cellule procaryote comme agent de transport ; et
    (b) un acide nucléique de peptide (PNA) à introduire dans le procaryote qui inhibe la transcription d'un gène procaryote qui confère une résistance contre les antibiotiques.

2. Conjugué selon la revendication 1, dans lequel le procaryote est une bactérie.

3. Conjugué selon la revendication 2, dans lequel la bactérie est une bactérie pathogène pour l'homme.

4. Conjugué selon l'une des revendications 1 à 3, dans lequel le peptide ou la protéine est un peptide ou une protéine antibactérien(ne).

5. Conjugué selon l'une des revendications 1 à 4, dans lequel l'agent de transport comprend une protéine phage-holine comportant l'une des séquences d'acides aminés représentées à la figure 3 ou un fragment ou une variante de celle-ci, qui peut traverser la membrane de cellule procaryote.

6. Conjugué selon l'une des revendications 1 à 4, dans lequel l'agent de transport comprend une défensine.

7. Conjugué selon l'une des revendications 1 à 6, dans lequel la résistance contre les antibiotiques est une résistance contre la pénicilline, contre l'ampicilline, contre la canamycine ou contre une tétracycline.

8. Conjugué selon l'une des revendications 1 à 7, présentant la structure suivante :

    agent de transport - espaceur - acide nucléique de peptide (PNA).

9. Conjugué ou mélange de conjugués selon la revendication 8, dans lequel l'espaceur est polylysine, polyglycine ou poly(glycine/lysine).

10. Conjugué selon la revendication 8 ou 9, dans lequel l'espaceur est couplé par l'intermédiaire d'un pont de disulfure fissile à l'agent de transport.

11. Conjugué selon l'une des revendications 6 à 10, dans lequel l'acide nucléique de peptide comprend la séquence $H_2N$-ATTGTTAGATTTCAT-COOH.

12. Médicament, qui contient un conjugué selon l'une des revendications 1 à 11.

13. Médicament selon la revendication 12, qui contient par ailleurs au moins un antibiotique pour lequel le procaryote a à nouveau été sensibilité par administration du conjugué.

14. Utilisation d'un conjugué selon l'une des revendications 1 à 11 ou de la composition définie à la revendication 13 pour la préparation d'un médicament pour traiter une infection procaryote.

15. Utilisation selon la revendication 14, dans laquelle l'infection procaryote est provoquée par un procaryote présentant une résistance contre au moins un antibiotique.

9

Strategien:

Def<sup>human</sup> -S⌒S- Glyc    PNA

→ spezifisch gegen **bakterielle Membranen**

+

speziell gegen **bakterielle Gene** gerichtete Anti-Gen-Strategie

→ Kombinierte 'Doppelstrategie'

Fig. 1

EP 1 471 943 B1

# Genom. Wirkort - Anti-Gen-PNA

DEFINITION   Cloning vector pBR322, complete genome.
ACCESSION    J01749  - Genbank
KEYWORDS     ampicillin resistance; beta-lactamase; cloning vector; drug
             resistance protein; origin of replication; plasmid;
SOURCE       Cloning vector pBR322.
ORGANISM     Cloning vector pBR322
             artificial sequence; vectors.


TTCTCATGTT TGACAGCTTA TCATCGATAA GCTTTAATGC GGTAGTTTAT CACAGTTAAA        60

TTGCTAACGC AGTCAGGCAC CGTGTATGAA ATCTAACAAT GCGCTCATCG TCATCCTCGG       120

CACCGTCACC CTGGATGCTG TAGGCATAGG CTTGGTTATG CCGGTACTGC CGGGCCTCTT       180
GCGGGATATC GTCCATTCCG ACAGCATCGC CAGTCACTAT GGCGTGCTGC TAGCGCTATA       240
TGCGTTGATG CAATTTCTAT GCGCACCCGT TCTCGGAGCA CTGTCCGACC GCTTTGGCCG       300
CCGCCCAGTC CTGCTCGCTT CGCTACTTGG AGCCACTATC GACTACGCGA TCATGGCGAC       360
CACACCCGTC CTGTGGATCC TCTACGCCGG ACGCATCGTG GCCGGCATCA CCGGCGCCAC       420
AGGTGCGGTT GCTGGCGCCT ATATCGCCGA CATCACCGAT GGGGAAGATC GGGCTCGCCA       480
CTTCGGGCTC ATGAGCGCTT GTTTCGGCGT GGGTATGGTG GCAGGCCCCG TGGCCGGGGG       540
ACTGTTGGGC GCCATCTCCT TGCATGCACC ATTCCTTGCG GCGGCGGTGC TCAACGGCCT       600

## Anti-Gen-PNA

## HOOC-TAC TTT AGA TTG TTA-NH₂

Fig. 2

EP 1 471 943 B1

## Alignments Holin-Protein (Phagen) - *Transportprotein*

product="probable holin" (GMSE-1)
[Endosymbiont bacteriophage may influence susceptibility to trypanosome infection in tsetse, Dale and Young]
protein_id="AAG50251.1"
db_xref="GI:12276078"
translation="MPCLIHLVGWGSSPGSALIREQAIGAGLAAWMTCLRGRYLGRGWRKTTFDAAICALIAWF
ARDGLALVGIDNQFSYLSSIIVGYLGNDYLGALLRRRLEKKS GESNAPQ

product="holin protein" (Listeria innocua)
protein_id="CAA61518.1"
translation="MMKMEFGKELLVYMTFLVVVTPVFVQAIKKTELIPSKWLPTVSILVGAILGALATSLDGSG
SLATMIWAGALAGAGGTGLFEQFTNRAKKYGKDD

product="holin" (bacteriophage 80 alpha)
specific_host="Staphylococcus aureus RN450
function="makes hole in membrane"
protein_id="AAB39698.1"
db_xref="GI:1763242"
translation="MDINWKLRFKNKAVLTGLVGALFVFIKQVTDLFGLDLSTQLNQASAIIGAILTLLTGIGVIT
DPTSKGVSDSSIAQTYQAPRDSKKEEQQVTWKSSQDSSLTPELSAKAPKEYDTSQPFTDASNDVGFDVN
EYHHGGGDNASKIN

product="holin" (Staphylococcus bacteriophage phi 11)
note="ORF3; structural homologue of holin"
protein_id="AAA99522.1"
db_xref="GI:511841"
translation="MDINWKLRFKNKAVLTGLVGALFVFIKQVTDLFGLDLSTQLNQASAIIGAILTLLTGIGVIT
DPTSKGVSDSSIAQTYQAPRDSKKEEQQVTWKSSQDSSLTPELSAKAPKEYDTSQPFTDASNDVGFDVN
EYHHGGGDNASKIN

product="putative holin 1" (Streptococcus pneumoniae bacteriophage MM1)
function="lysis protein"
protein_id="CAC48114.1"
db_xref="GI:15074937"
translation="MKIEFFNFLRSVIQTEDGLVLYALALIVSMEIIDFVTGTIAAIINPDIEYKSKIGINGLLRKISGV
LLLMILIPASVLLPEKTGFAFLYSICLGYIAFTFQSLIENYRKLKGNVTLFQPIVKVFQRLLEKDDDTKKGE

gene="orf87a" (Streptococcus thermophilus bacteriophage Sfi21)
product="holin"
protein_id="CAA64941.1"
db_xref="GI:2292749"
translation="MKKRKKKMINFKLRLQNKATLVALISAVFLMLQQFGLHVPNNIQGINTLVGILVILGIITDP
TTKGIADSERALSYIQPLDDKEVY

gene="hol500" (Bacteriophage A500);(Listeria monocytogenes)
protein_id="CAA59363.1"
/db_xref="GI:853745"
/translation="MMKMEFGKELLVYMTFLVVVTPVFVQAIKKTELIPSKWLPTVSILVGAILGALATSLDGSG
SLATMIWAGALAGAGGTGLFEQFTNRAKKYGKDDK

product="holin" (Bacteriophage PL-1)
protein_id="BAA96748.1"
translation="MQNELLQVLAIAFVIAPITTGFTEIFKRYTPAEGKLLPVLSIGTG
ILLACVWAMAFGHLPLIGAYALAGMLSGLASVGVYQIVKPNEEVK

Fig. 3(1)

gene="lydA" (Bacteriophage P1) (enterobacteriae)
codon_start=1
product="holin"
protein_id="CAA61014.1"
db_xref="GI:974764"
translation="MLDTQELAPVAIALLLSVIGGIGTFLMDVRDGRQSGNLLGLVTEIFVAVTAGAVAYLLGQH
EGWELSITYLMVTIASNNGHEVISGMKRVNIDSILNVLTSL VKKGGGK

gene="S" (Bacteriophage H-19B)
note="similar to Bacteriophage 21 lysis gene S, encoded by GenBank Accession Number M65239" /
product="putative holin protein"
protein_id="AAD04658.1"
db_xref="GI:2668771"
translation="MEKITTGVSYTTSAVGTGYWLLQLLDKVSPSQWVAIGVLGSLLFGLLTYLTNLYFKIREDR
RKAVRGE

gene="hol" (Bacteriophage A118)
function="forms unspecific lesions into cytoplasmic membrane prior to lysis"
specific_host="Listeria monocytogenes"
note="ORF24; two products may be translated from this gene (hol-96 and hol-93)"
product="holin"
protein_id="CAB53810.1"
db_xref="GI:5823622"
translation="MIEMEFGKELLVYMTFLVVVTPVFVQAIKKTELVPSKWLPTVSILIGAILGALATFLDGSGS
LATMIWAGALAGAGGTGLFEQFTNRSKKYGEDDK

gene="Hol" (Lactobacillus casei bacteriophage A2)
product="putative holine"
protein_id="CAB87385.1"
db_xref="GI:7573220"
translation="MKINWKVAVLSVKFWLALVPAALLVVQTAAAVFGYNWDFANLGKELTAVINAVFALLTI
VGVAVDPTTEGVSDSQQALAYPALITTKAAKIKSLEDQIKALQADKAADQATSAASEVVPETSSAAPAE
SAPESVAPVASEEVK

gene="Hol" (Lactobacillus bacteriophage phig1e)
product="holin"
protein_id="CAA66751.1"
db_xref="GI:1926366"
translation="MDIITSLNLATAGELALISFFIGVIVQAIKKTGKVKNTYLPFISMGIGILAGLAAVVVTKDTN
YLNGAVAGLIVGAATSGLTDGLSVGTSAVTTAKATKDAAKTAAITQAVLNSINTTKSSDTTQVANTSN
TEGGSTSETQK

product="holin" (Lactobacillus delbrueckii subsp. lactis bacteriophage LL-H)
protein_id="AAC00556.1"
db_xref="GI:623083"
translation="MTLIDWFNLIVAIGTIALAVVASVYVHLKAKIDTKTAAGKAFDLVGKLAVWAVNEAEHSQ
DGGAAKREFAAKLISDQLKAKGITGIDEKMVYGAVETAWKEA IENVK

product="holin protein" (Lactococcus phage c2)
protein_id="AAD20611.1"
db_xref="GI:4426933"
translation="MIETLRAIGLVVFMQLLSLALEFIDTGTLKPSVRKRIAVELMVL

gene="hol" (bacteriophage phiAM2)
note="hydrophobic pore-forming protein"
product="holin"
protein_id="AAG24367.1"
db_xref="GI:10880732"
translation="MFFNNKFYNVIKWAVLTALPALSVFIGVIGKAYGWGGTDLAIITLNAFTVFLGTLAGVSAV
KYNSQPNDTKENK

Fig. 3(2)

product="holin"
protein_id="AAG24367.1"
translation="MFFNNKFYNVIKWAVLTALPALSVFIGVIGKAYGWGGTDLAIITLNAFTVFLGTLAGVSAV
KYNSQPNDTKENK

product="holin" (Bacteriophage Tuc2009)
protein_id="AAA32614.1"
db_xref="GI:496282"
translation="MNQINWKLRLKSKAFWLALLPALFLLIQAIGAPFGYKWDFVILNQQLAAVVNAAFALLAI
VGVVADPTTSGLGDSDRVLNKDKSEENK

product="holin" (Bacteriophage TPW22)
function="formation of non-specific lesions in the cytoplasmic membrane"
protein_id="AAF12704.1"
db_xref="GI:6465904"
translation="MNQINWKLRLKSKAFWLALLPALFLLIQAIGASFGYKWNFVILNQQLAAVVNAAFALLAI
VGVVADPTTSGLGDSDRVLNKDKSEENK

product="holin" (homology to Orf78 of phage HP1 and gene S of phage P21)
protein_id="AAC45168.1"
db_xref="GI:915370"
translation="MRFNMLKNSETTGAYVGSAIAIYSGFTLADWAAIFGILFGLFT M LINWYYKNK
EIKLKETALKQKIDLKEGDHE

product="holin" (Bacillus phage GA-1)
function="host cell lysis, holin formation"
protein_id="CAC21535.1"
db_xref="GI:12141291"
translation="MFEFFHSLMETDDTKVYFLLGIIGVLNIVDFFFGFINAKFNKSIAYKSSKTIDGIMRKMKFTI
MAILFIPVSVLMPEPIGLGALYVFYFGYIYAELNSILSH LKLSEDGKETEVFLDFINTFFNSTKGDKKDD

gene="hol187" (Staphylococcus phage 187)
function="forms pores to allow access of lysin to CW"
product="holin protein Hol187"
protein_id="CAA69023.1"
db_xref="GI:2764984"
translation="MLMVIMVGNVGIYLTIFLIDTGTLRHQATQEIWHGIDILKGLKC LETLLILSLNQVI

gene="s" /function="holin" (Shigella dysenteriae)
product="S protein"
protein_id="CAC05628.1"
db_xref="GI:9955825"
translation="MYQMEKITTGVSYTTSAVGMGYWFLQFLDRVSPSQWAAIGVLGSLLFGLLTYLTNLYFKI
REDRRKAARGE

gene="E"
protein_id="CAA42879.1"
db_xref="GI:14781"
db_xref="SWISS-PROT:P31280"
translation="MERWTLLDILAFLLLLSLLLPSLLIMFIPSMYKQHASLWKARSLAKTLSMASSARLTPLSSS
RTPCVLKQDSKKL

gene="xhlB" (B.subtilis DNA (28 kb PBSX/skin element region)
product="holin-like protein"
protein_id="CAA94048.1"
db_xref="GI:1225964"
db_xref="SWISS-PROT:Q99163"
translation="MNTFDKGTVIRTVLLLIALINQTMLMLGKSPLDIQEEQVNQLADALYSAGSIAFTIGTTLAA
WFKNNYVTEKGKKQRDLLRDNNLTK

Fig. 3(3)

gene="bhlA" (Bacillus subtilis 168 prophage)
product="holin-like protein"
protein_id="AAC38301.1"
db_xref="GI:2997596"
translation="MEMDITQYLSTQGPFAVLFCWLLFYVMKTSKERESKLYNQIDSQNEVLGKFSEKYDVVIE
KLDKIEQNFK

gene="bhlB" (Bacillus subtilis 168 prophage)
product="holin-like protein"
protein_id="AAC38302.1"
db_xref="GI:2997597"
translation="MFENIDKGTIVRTLLLAIALLNQIMVMLGKAAFIINEEDINHLYDCLYTIFTIVFTTSTTTAA
WFKNNYITAKGKKQKQVLKKENLFK

gene="hol" (Bacteriophage phi-Ea1h)
specific_host="Erwinia amylovora
function="pore formation"
product="holin"
protein_id="CAC17008.1"
db_xref="GI:11342496"
translation="MRKIYVVIITTIVVAGLIWAFIATQVNTGVTSKRQEDALAVSEANVGIGKEAKDQGEQATK
RADVAKEQRTHQINQLKDKLHEKAESYDSIPLSPSDVDILC RAYRSTDPVCSPTVKSD

Fig. 3(4)

Alignments **Lysis-Protein** (Phagen)

product="lysis protein" (Phage phiX174)
function="host cell lysis"
protein_id="CAA84691.1"
translation="MVRWTLWDTLAFLLLLSLLLPSLLIMFIPSTFKRPVSSWKALNLRKTLLMASSVRLKPLNCS
RLPCVYAQETLTFLLTQKKTCVKNYVQKE

Fig. 3(5)

# Fig. 4

I  /  E. Coli$_{competent}$ XL - 10 GOLD  + PNA-Probe (500 nM)

KANAMYC-Dishes

1 µl = 25 µM PNA*

AMPICILL- Dishes

10 µl = 2.5 µM PNA*

NaCl-Control          PNA*-Probe

Fig. 4

* Peptid-Nukleinsäure-Transport-Komple

EP 1 471 943 B1

intakte Bakt. + Inkubation mit α-Kan-PNA

E.K.PNA-Konstr. ≈ 2 μM

≈ 2 μM

Fig. 5

E. Coli<sub>intact/Kanresist</sub>

Optimizing Number of E. Coli per plate
serial dilution of E. Coli by spectrophotometric measurements 600 nm

•1) 1 : 10
•2) 1 : 100
○3 1: 1000
○4) 1: 10000
○5) 1: 100000
○6) 1: 1000000

Serial Dilution of intact E. Coli to optimize the antibacterial incubation   Fig. 6

* Peptid-Nukleinsäure-Transport-Komp

III     K1-Control E. Coli$_{intact/Kanresist}$

Kanamycine Dishes

3    PNA$_{/Ampresistgene}$ (2,5 µM) - 10 µl     2    PNA$_{/Kanresistgene}$ (25 µM) - 1 µl

Identic: Bacter. Nmb; identic. PNA conc

Fig. 7

IV / analogue III - K1-Control E. Coli$_{intact/Kanresist}$

4   PNA$_{/Ampresistgene}$ (2,5 µM) - 10 µl
1µl E. Coli + 10 10 µl PNA
in LB medium

Fig 8

* Peptid-Nukleinsäure-Transport-Kompl

2: PNA 0,25 μM

1: PNA 2,5μM

Fig. 9

EP 1 471 943 B1

Fig. 10

# Test for NON-Toxicity in eucaryotic cells (HeLa)

HeLa
Control ———→
(untreated)

*A*

PNA*<sub>AMPRESGENE</sub> - 25 µM

PNA*<sub>KANRESGENE</sub> - 25 µM

*B*

Fig. 11

* **Peptid-Nukleinsäure-Transport-Kompl|**

EP 1 471 943 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19933492

**In der Beschreibung aufgeführte Nicht-Patentliteratur**

- **GOOD et al.** Bactericial antisense effects of peptide-PNA conjugates. *Nature Biotechnology,* 2001, vol. 19 (4), 360-364
- **MERRIFIELD.** *J. Am. Chem. Soc.,* 1963, vol. 85, 2149
- **SAMBROOK et al.** Molecular Cloning: A Laboratory Manual. Cold Spring Harbor Laboratory Press, 1989
- **TANG et al.** *Science,* 1999, vol. 286, 498
- **SAIDO-SAKANAKA et al.** *Biochem. J.,* 1999, vol. 338, 29
- **RAJ et al.** *Biochem J.,* 2000, vol. 347, 633
- **YU et al.** *J. Biol. Chem.,* 2000, vol. 275 (6), 3943
- **NIELSEN et al.** *Science,* 1991, vol. 254, 1497-1500
- **MERRIFIELD.** *J.Amer.Chem.Soc.,* 1963, vol. 85, 2149-2154
- **RUEGG ; RUDINGER.** *Methods Enzymol.,* 1977, vol. 47, 111-126